# EUROPEAN PATENT SPECIFICATION

(11) **EP 1 667 699 B1**
(45) Date of publication and mention of the grant of the patent: **18.08.2010**
(21) Application number: 03759697.0
(22) Date of filing: 03.10.2003
(51) Int. Cl.: A61K 8/97, A61Q 19/08, A61K 36/62

(54) **Compositions comprising lotus extracts and methyl donors**
Zubereitungen die Lotusextrakte und Methyldonatoren enthalten
Compositions contenant des extraits de lotus et des donneurs de méthyle

(43) Date of publication of application: 14.06.2006
(73) Proprietor: Green Meadows Research. LLC., Dublin 1 IE (IE)
(72) Inventor: RILEY, Patricia, A., Golden Beach, FL 33160 (US)
(74) Representative: Senior, Janet
(86) International application number: PCT/US2003/031392
(87) International publication number: WO 2005/041996

(56) References cited:
- WO-A-96/12797
- US-A- 5 925 348
- US-A1- 2002 098 253
- US-A1- 2003 091 665
- US-B1- 6 468 564

## Description

### FIELD OF THE INVENTION

The present invention relates to the use of the Lotus plant and especially Lotus extract in oral and topical applications. In addition, the present invention concerns the addition of methyl donors and other antioxidants to the formulation to protect the body from free radicals, a key factor contributing to aging.

### BACKGROUND

The human body is assaulted on a daily basis by factors that accelerate the aging process - diet, stress, sun, heat, environment, and the formation of free radicals, which damage the skin cell membrane and DNA by various pathways.

The free radicals originate from many reactions. For example, molecular oxygen can be stripped of an electron by photons of light (solar radiation), heat, or an oxidant. Thus formed, singlet oxygen is highly reactive and short lived; it quickly adds another electron, producing the super oxide anion radical and the hydroxyl radical. All of these radicals are toxic to cells and highly reactive, causing a cascade of damage that accumulates over the years and contributes to aging.

In addition, free radicals are produced by normal metabolism, where oxygen is used to convert food for energy, as well as in certain diseases, such as infection and inflammation, in which the immune cells use free radicals, such as nitric oxide and superoxide radicals, to destroy bacteria and viruses. However, these free radicals, if in excess, can produce more damage, in particular to DNA, causing mutations and fragmentations.

The damage doesn't stop at the cell or molecule that is oxidized, since the oxidized component within the cell or molecule becomes unstable and highly reactive due to its need for an electron, it becomes a free radical attacking other cells or molecules, thereby causing a chain reaction.

For general health, oxidation of lipids and proteins injures cell membranes, weakens blood vessels, modifies enzymes and damages many other molecules. These injuries alter cell functions and increase the risk of heart disease, stroke, and cancers.

The effects of free radicals on the skin is reflected in accelerated aging; that is, a loss of tone and elasticity and formation of wrinkles and lines. These effects are due to the damage free radicals have on collagen levels and chemical changes to the collagen molecule itself by various pathways.

In addition to the aging factors, as discussed above, skin is also subject to other aging factors. Within the dermis are highly stable fibers of collagen and elastin. Collagen, the most abundant protein in the body, has a high tensile strength thus preventing skin from being tom by over stretching. Elastin, also a protein, allows movement. As skin ages, elastic tissue increases until it loses the ability to stretch and recover. This loss of resiliency and elasticity is accompanied by increased stiffness, sagging and wrinkling. Changes in collagen solubility and cross-linking contribute to loss of elasticity. Cell renewal, skin thickness and circulation decrease with aging, affecting the skin's ability to withstand stress from UV radiation and free radicals.

On the cellular level, aspartyl and asparaginyl residues are prominent sites of age-related damage in proteins. These damaged sites have been characterized in a variety of proteins, but are particularly common in the long-lived proteins. Enzymatic mechanisms for reversing damage to DNA are well established and have been shown to be essential for extended lifespan.

Experiments performed *in vitro* with recombinant and chemically modified polypeptides have shown that the presence of an L-isoaspartyl residue may alter both enzymatic activity and the binding of other molecules.

Limiting the accumulation of these residues within cells is currently believed to be important; all human cells examined thus far contain an L-isoaspartyl/D-aspartyl protein methyltransferase that has been proposed to serve this function. It is also believed that this methyltransferase can recognize both D-aspartyl and L-isoaspartyl residues. In addition, it is thought that this enzyme may have the ability to reverse at least part of the damage to protein molecules.

Although the human isoaspartyl protein repair methyltransferase has been purified from red blood cells and had its protein sequence determined, in addition to harvesting a variant in a bacterial system, the availability and use of methyltransferase has been limited.

On or around November 14, 1995, however, it was reported that scientists germinated a 1,288 year old Sacred Lotus seed. The research reported in the November issue of the American Journal of Botany, began in 1982, when Jane Shen-Miller, a plant physiologist at the University of California at Los Angeles (UCLA) obtained seven brown, oval-shaped Sacred Lotus seeds from the Beijing Institute of Botany.

In 1983, Jane Shen-Miller filed through the hard shells of four of the ancient Sacred Lotus seeds and watched three of them sprout. She then dried and burned the seedlings so she could use radiocarbon dating to establish the ages, the oldest of which was 1,288 years old. It has been postulated as a result of this research that Sacred Lotus seeds act as live embryos until such seeds are germinated. Up until this point, geneticists knew only about proteins that repaired damaged DNA. But findings have suggested that the L-isoaspartyl methyltransferase (MT) enzyme, found in the Sacred Lotus seeds and nearly all other organisms, may have the ability to repair other proteins - those that make up cells and tissues, thus slowing tissue decay.

In these ancient Sacred Lotus seeds, the MT enzyme was present at levels comparable to modern day Sacred Lotus seeds. Damaged proteins did not accumulate within the ancient Sacred Lotus seeds, suggesting that the MT enzyme, possibly along with other constituents, such as methyl donors and antioxidants, kept the ancient Sacred Lotus seeds alive for so many years.

Notwithstanding the above, it is unknown as to whether use of methyltransferase or extracts or components of the Sacred Lotus plant in topical or oral compositions would be effective in combating aging, repairing damaged skin and/or restoring skin to a more youthful appearance. Moreover, there are no known acceptable products available which incorporate methyltransferase or extracts or components of the Sacred Lotus for combating dermatological aging, repairing damaged skin and/or restoring skin to a more youthful appearance. US-6468564, US-20021009853 and US-5925348 disclose anti-aging compositions comprising Lotus seed extract and methyltransferase.

### SUMMARY OF THE INVENTION

The present invention alleviates and overcomes certain of the above-mentioned problems and shortcomings of the present state of the art through the discovery of novel acceptable oral and topical delivery systems and compositions for effectively treating and preventing signs of aging, repairing damaged skin and restoring skin to a more youthful appearance and methods of using the same.

Accordingly it is the object of the invention to provide a general method for prevention or alleviation of age-related damage to the skin and associated decline of body function and appearance from aging through the topical and/or Internal use of a composition in combination with a suitable carrier or vehicle,

Another object is to restore the skin to a more youthful appearance.

Another object is the formulation of dietary supplements to nourish and protect skin from within.

Another object is to have the person taking the supplement experience improvement in physical attributes helping the person to feel younger and look younger; have increased stamina; have increased sexual function; have improved circulation; have improved hair and nail strength and growth; have improved vision; have improved mental focus, performance and memory; have improved overall health; have improved immune function: have improved body composition, with greater muscle tone; have improved body composition, with less body fat; have improved ability to sleep; have improved vitality; have improved energy; have improved mood; restore skin to a more youthful appearance; improve the vibrancy of skin; improve the smoothness of skin; improve the tone of skin; improve the elasticity of skin; and have less wrinkles.

These and other objects are achieved by the present invention which is directed to a topical or oral formulation for the protection of the skin against free radical damage contributing to aging and a method for preventing or alleviating such damage and restoring skin to a more youthful appearance by employing such in a topical or oral formulation.

The present inventor has been experimenting with the use of Lotus seed extract in oral and topical formulations since 1996. Ongoing research has revealed that colorful fruits and vegetables, such as carrots and broccoli, contain excellent antioxidant properties, As evidenced by their names (Blue Lotus, Yellow Lotus, Purple Lotus, etc.), the flowers of the *Nelumbo* family are also very colorful. The present inventor realized that the pigments contained in the Lotus flower would act similarly to antioxidants and that the combination of Lotus seed extract and Lotus flower extract may prevent free radical damage and extend the youthfulness of the skin.

It has now been observed, surprisingly and unexpectedly, that by using an extract of Lotus in combination with methyl donors by topical application or oral ingestion according to claim 1, signs of aging can be reduced, eliminated, or even reversed.

Thus, the present invention provides an oral or topical composition comprising:
a) Lotus seed extract;
b) Lotus flower extract;
c) one or more methyl donors; and
d) a compatible vehicle for oral or topical application.

Until the present invention, there were no acceptable vehicles utilizing Lotus extract in combination with synergistic acting methyl donors in an elegant cosmetic and/or basic pharmaceutical composition. According to the present invention, formulations of Lotus extract in combination with methyl donors may be used in dermatologicals, such as gels, lotions, powders, tablets, capsules, creams, sunscreens, cleansers, and various skin care formulas to repair the damage from aging, reduce further damage and restore skin to a more youthful appearance. Another object of the present invention is to add Lotus extract and methyl donors to existing preparations to reduces the appearances of lines and wrinkles.

A formulation of the invention comprises a suitable cosmetic or dermatologically or orally acceptable non-toxic, non-allergenic carrier containing a variety of components.

In one embodiment a cosmetic composition is enhanced to make the skin appear younger by adding methyl donors and, as a natural source of methyltransferase, Sacred Lotus (*Nelumbo nucifera*).

The above features and advantages of the present invention will be better understood with reference to the detailed description.

### DETAILED DESCRIPTION

The novel topical and oral delivery systems of the invention contain a combination of Lotus extract and methyl donors according to claim 1 for effectively treating and preventing aging, repairing damaged skin and restoring skin to a more youthful appearance and improving overall health and methods of using same.

The present invention uses Lotus extract in skin and dietary formulas to combat aging. It is believed that the use of Lotus extract in cosmetics and dietary supplements accomplishes anti-aging effects based on anti-aging factors (enzymes such as methyltransferase) which are present in the Lotus plants and seeds.

Moreover, Lotus extracts contain certain anti-oxidants such as vitamin C and glutathione which may contribute to anti-aging effects on skin along with methyltransferase and may even contain other beneficial factors. Additional antioxidants may be used to counter the deleterious effects of free radicals.

In accordance with the present invention, methyltransferase and/or the natural compounds found in an extract of Lotus plants may be used in an effort to repair age related signs of the skin such as lines, spots, wrinkles, and/or loss of elasticity. Other sources of methyltransferase that may be used in the invention are extracts of Sacred Lotus seeds or other components of the Sacred Lotus plants, extracts of Yellow Lotus seeds or components of Yellow Lotus plants, extracts of Purple Lotus seeds or other components of Purple Lotus plants, extracts of Blue Lotus seeds or other components of Blue Lotus plants, extracts of White Lotus seeds or other components of White Lotus plants, extracts of other Lotus seeds or components of other Lotus plants where methyltransferase may be present, wheat germ oil, liver, brain, other animal parts including glands, and bio-fermentation or chemical synthesis for example.

An extract is prepared, for example, as follows. Maceration is the preferred process, since no heat is used which may destroy or alter temperature sensitive components; however percolation, digestion, infusion and decoction are within the scope of the invention as more scientific information becomes available.

A twenty percent extract of *Nelumbo nucifera* is used and prepared by placing 200 grams of finely milled untreated whole seeds, including husks and piths, in a stoppered container with about 750 mL of a 50/50 wt/wt mixture of purified water and propylene glycol USP and allowed to stand for a period of at least three days in a warm place with frequent agitation, until soluble matter is dissolved. The mixture is filtered and, after most of the liquid has drained, the residue on the filter is washed with sufficient quantity of solvent mixture; the filtrates are combined to produce 1000 mL.

It is also within the scope of the present invention to use different amounts of the seed, other parts of the plant, as well as other specles, solvents and mixtures.

While it is believed that Sacred Lotus (*Nelumbo nucifera*) is a preferred species and the seed and flower are the preferred parts, other parts of the plant or other species, such as the Yellow Lotus (*Nelumbo lutes*). Blue Lotus (*Nelumbo caerulea*), White Lotus or other Lotus varieties containing these anti-aging enzymes or constituents are believed to be suitable alternatives for accomplishing the objectives of the present invention.

Tne oral or topical compositions can comprise an extract of the seed of plant material selected from the group consisting of the Yellow Lotus (*Nelumbo lutea*), the Blue Lotus (*Nelumbo caerulea*) and the Sacred Lotus (*Nelumbo nucifera*).

As discussed above, methyltransferase and/or the natural compounds found in the extract of the Lotus is used to combat the natural aging process. However, the skin is also subject to external factors that contribute to accelerating the aging process. The present invention combats these external factors utilizing extracts of the Lotus in combination with methyl donors. It is believed that this mixture provides added protection from external factors that damage the skin.

The present invention combines Lotus extract with methyl donors. As discussed above, it is believed that Lotus extract contains the L-isoaspartyl methyltransferase enzyme. In general, the methyltransferase enzyme catalyzes the transfer of a methyl group to an acceptor molecule. Many different methyttransferase redactions have been documented in the literature, including 1-phenanthrol methyltransferase activity, 5, 10-methylenetetrahydrofolate-dependent methyltransferase activity, 5-methyl-5,6,7,8-tetrahydromethanopterin-dependent methyltransferase activity, to name a few. According to the theory of the present invention, the Lotus extract contains the L-isoaspartyl methyltransferase enzyme, which transfers a methyl group to damaged L-isoaspartyl residues. This mechanism serves to repair cell damage to all human cells.

As discussed above, Applicant has discovered that the addition of methyl donors to topical and oral compositions containing Lotus extract results in improved skin benefits by the repair of age-related damage in proteins by limiting the accumulation of L-isoaspartyl residues. Methyl donors include any compounds that have a methyl (-CH₃) side chain. Some suitable examples include methionine, betain (aka trimethylglycine), dimethylglycine, choline, polyenyphosphatidylcholine, phosphatidylserine, phosphatidyl choline (lecithin), glycerylphosphoryl choline, and S-sdenosyl-L-methionine ("SAMe"). The methyl donor provides the methyl group needed by the L-isoaspartyl methyltransferase to repair the damaged cell.

The present invention uses a combination of Lotus extract and methyl donors according to claim 1 in oral and topical formulations. It is theorized that this combination may act in concert with or perhaps synergistically with one or more of the following ingredients:

### Vitamins

Any known vitamins or their derivatives are useful in the present invention. Vitamins, by definition, are any of a group of organic substances essential in small quantities to normal metabolism. Synthetic vitamins are also available and may be utilized in the present invention. It is believed that the addition of one or more vitamins to the proposed combination would benefit the end user by providing components that may be needed in the cell repair process.

Vitamin A, or retinol, is a fat soluble vitamin. Vitamin A has been demonstrated to aid bone development, strengthen the immune system and promote wound healing. Sources of Vitamin A include broccoli, cantaloupe, cod, halibut, kale, red peppers, spinach and watercress. The addition of natural or synthetic Vitamin A and derivatives thereof to the present invention would prove beneficial.

Vitamin B-1, or thiamine, is a water soluble vitamin. Vitamin B-1 has been demonstrated to assist in the metabolism of proteins and fats. Sources of Vitamin B-1 include chick peas, pinto beans, soybeans, nuts, and salmon.

Vitamin B-2, or riboflavin, is a water soluble vitamin. Vitamin B-2 is essential for normal cell growth. Sources of Vitamin B-2 include almonds, walnuts and dairy products, such as cottage cheese, milk and yogurt. The addition of natural or synthetic Vitamin B-2 and derivatives thereof to the present invention would prove beneficial.

Vitamin B-3, or niacin, is a water-soluble vitamin. Vitamin B-3 has been demonstrated to protect against carcinogens and is useful in maintaining healthy skin. Sources of Vitamin B-3 include almonds, sunflower seeds, hazelnuts and yogurt.

Vitamin B-6, or pyridoxine, is a water-soluble vitamin. Vitamin B-6 is essential for the proper functioning of over sixty enzymes. Sources of Vitamin B-6 include eggs, salmon, pinto beans and lentils.

Vitamin B-12, or cyanocobalamin, is a water-soluble vitamin. Vitamin B12 helps maintain healthy cells. Sources of Vitamin B-12 include eggs, yogurt, salmon and halibut.

Vitamin C, or ascorbic acid, is one of several antioxidants shown to be essential for reducing free-radical induced cellular and molecular events when skin or cells are exposed to substantially high doses of ultraviolet B radiation. Vitamin C has also been shown to inhibit ultraviolet radiation-induced immunosuppression *in vitro.* Sources of Vitamin C include broccoli, cantaloupe, citrus, red peppers, strawberries and tomatoes.

Vitamin D is a fat soluble vitamin. Vitamin D is essential in calcium absorption and metabolism. Sources of Vitamin D include fish, such as cod.

Vitamin E is the α-form of tocopherol. There are four stereoisomers of tocopherol and four stereoisomers of tocotrienol. The tocopherols and the tocotrienols both contain a central aromatic chromanol nucleus and a 16-carbon member side chain. The side chain in the tocopherols is saturated. The side chain in the tocotrienols is not. Vitamin E is found in palm oil, soy bean oil, com oil, canola oil, cranberry oil, rapeseed oil, rice bran oil, sunflower oil, safflower oil and cottonseed oil. However, of these, only cranberry oil, palm oil and rice bran oil contain tocotrienols. α-tocopherol is one of several antioxidants shown to be essential for reducing free-radical induced cellular and molecular events when skin or cells are exposed to substantially high doses of ultraviolet B radiation. α-tocopherol has also been shown to inhibit ultraviolet radiation-induced immunosuppression *in vitro.* Sources of Vitamin E include almonds, hazelnuts, pecans, sunflower seeds, asparagus, olives, and spinach.

Vitamin F, also known as Essential Fatty Acids or EFA, is a fat-soluble vitamin. Vitamin F consists of unsaturated fatty acids with two or more double bonds, such as linoleic acid and linolenic acid. The term Vitamin F was discredited by the American Medical Association in 1937. Sources of Vitamin F include vegetable oils, such as olive oil.

Vitamin H, also known as biotin, is a water soluble vitamin. Sources of biotin include liver, kidney, pancreas, yeast and milk.

Vitamin K is a general term that refers to a group of naphthoquinone derivatives required for the bioactivation of proteins involved in blood coagulation or stagnation. Sources of Vitamin K include green plants and intestinal bacteria.

### Minerals

Any known minerals or their derivatives are useful in the present invention. There are several definitions for minerals, including, "any substance that is neither animal nor vegetable." However, the use of the term in the present invention is directed to any of a group of in organic substances essential to the functioning of the human body and that are usually obtained from foods. It is believed that the addition of one or more minerals to the proposed combination would benefit the end user by providing components that may be needed in the cell repair process.

Minerals useful in the present invention include copper, zinc, and manganese, for their role in forming the antioxidant, superoxide dismutase, and selenium and glutathione, for their role in function of glutathione peroxidase. Other minerals involved in cell function maintenance include calcium, magnesium and potassium. Other useful minerals include sulfur, chromium, iron, phosphorous and iodine.

### Antioxidants

Antioxidants protect skin cells and collagen from free radical damage. The present invention may combine Lotus and methyl donors with antioxidants to provide stronger damage protection. Some of the antioxidants useful in the present invention include glycyrrhiza glabra, quercetin, rosemary, sage, ginger, tumeric, carnosine, curcumin, limonene, silymarin, isoflavones, carotenoids, asaxanthin, lycopene, lutein, superoxide dismutase, aloe vera, carnosine, CoEnzyme Q10, hawthorne berry, hawthorne berry extract, acerola, acerola extract, apricot, apricot extract, bilberry, bilberry extract, blackberry, blackberry extract, black currant, black currant extract, blueberry, blueberry extract, chaste berry, chaste berry extract, cherry, cherry extract, cranberry, cranberry extract, elderberry, elderberry extract, mulberry, mulberry extract, raspberry, raspberry extract, strawberry, strawberry extract, ubiquinone, acerola berry, olive pulp extract, including hydroxytyrosol, broccoli sprouts, ginko biloba, ginseng, carnitine, pycnogenol, oregano, inositol, choline, willow bark extract, acetylsalicylic acid, meadowsweet flower, and DMAE (dimethylamino ethanol). The berries listed above can be used as the fruit, as a powder or as an extract.

### Amino Acids

Amino acids, such as glycine, glutamine, proline, and lysine, are components of collagen and therefore useful in compositions of the present invention. Valine, arginine, and alanine are vital to skin repair. Collagen is one part of connective tissue and the major component of the skin. 4-hydroxyproline is found mainly in collagen. Serine is a non-essential amino acid for human development.

### Hormones

Hormones are vital to the skin's natural metabolism. With age, hormone levels decline in the body and the skin. Cell function declines as hormonal levels drop. It is known that estrogen and testosterone can restore some youthfulness to the skin's appearance. This invention encompasses these and other compounds with hormonal activity that rejuvenate the skin's youthful appearance. Some of the hormones useful in the present invention include estrogen and its components (estradiol, estriol and estrone), testosterone, pregnenolone, progesterone, melatonin, and DHEA. Phytoestrogens from plants are also useful in the present invention and include soy and its constituents, yam, polygonum cuspidatum, black cohosh, licorice, grapes/resveratrol, dongquai, red clover, and vitex agnus castus.

Growth factors, such as EGF (Epidermal Growth Factor), FGF (Fibroblast Growth Factor) or IGF (Insulin-like Growth Factor), appear to act in concert with or perhaps synergistically with Lotus.

### Compounds commonly used in cosmetics as skin beautifiers

The present invention may also include the following conventional ingredients:
α - hydroxy acids, also known as fruit acids, are well-known skin rejuvenating agents for photoaged and aged skin. Some examples of α - hydroxy acids include citric, glycolic (sugar cane extract) and lactic acids.

Glycerin is a common humectant in both topical and oral formulations. It is used to dissolve non-water soluble substances. It is also used to provide a moisture barrier between the skin and the environment.

Rice bran oil, walnut oil, almond oil, pumpkin seed oil, sunflower seed oil, safflower seed oil, fish oil, omega 3 fatty acids, omega 6 fatty acids, DNA/RNA, vitamin D, policosanol,

The term surfactant is used to describe an additional group of additives to the present invention. Surfactants are solubilizers which are used to promote solubility. Surfactants for use in the present invention are pharmaceutically acceptable and include polysorbates and partial esters of common fatty acids, to name two of many available.

Sunscreen agents are also within the scope of the present invention. Titanium dioxide is a well-known cosmetic ingredient used topically to protect the skin from photo-aging. Octylmethoxycinnamate, benzophenone, octylsalicylate, and parsol 1789 can also be used in combination with Lotus and/or methyl donors to increase the skin's defense against sun damage.

It should be understood that the topical or oral compositions of the present invention may be used at any appropriate daily intervals, depending of course upon the particular type of composition formulated. For instance, the Firming Antioxidant Serum, as set forth in Example 2 hereinafter, is to be used anytime when desired, i.e., at night, under make-up, by itself, or before using or with other preparations. Whereas anyone skilled in the art, obviously can formulate a myriad of products for special needs that would be within the scope of this invention.

A composition according to the present invention may be in any of the cosmetic, pharmaceutical or dietary forms which are generally used for topical application such as liquids (both aqueous and non-aqueous solutions), creams (both oil-in-water and water-in-oil, O/W & W/O, emulsions), gels (both aqueous and non-aqueous), lotions, serums, ointments, paste, powders, liposomes, laminates, microspheres, capsules, and tablets.

Compositions of the present invention may also contain additives such as water, alcohols, oils (mineral vegetable, animal and synthetics), glycols, colorants, preservatives, emulsifiers, gelling agents, gums, esters, silicones, polymers, fragrances, flavors, active ingredients, acids, bases, buffers, salts, polyols, proteins and their derivatives, essential oils, tonics, waxes, lipids, stabilizers, fillers, celluloses, glycans, amines, solubilizers, thickeners, sugars and sugar derivatives, ceramides, sweeteners and the like, so long as such additives do not defeat the objectives of the present invention.

Other items such as lecithin, squalene, panthenol, jojoba oil, vegetable oils, saccharide isomerate, glycerin, dimethicone, aloe, saccharide isomerate and other moisturizers also appear to benefit the present invention In holding moisture In the skin, enhancing its repair process, protecting the skin barrier function and making the skin appear younger.

The following examples are given for illustrative purposes only to delineate some of the features of the invention. The quantities are given in percent weight (%wt) or international units (I.U.), unless otherwise noted, based on the total weight of the composition. The term qs means to use a sufficient quantity by weight to bring the entire composition to 100%. Whenever possible, International Nomenclature Cosmetic Ingredient (INCI) names are used.

### Reference Example 1

### DAILY NUTRIENTS

| | | |
|---|---|---|
| Vitamin A Palmitate | 1750 | IU |
| Natural Beta Carotene | 750 | IU |
| Lycopene | 0.375 | mg |
| Lutein | 0.375 | mg |
| Vitamin E (d-alpha tocopheryl acetate and | | |
| mixed tocopherols and tocotrienols) | 50 | IU |
| Cholecalciferol (Vitamin D3) | 200 | IU |
| Vitamin C | 150 | mg |
| Thiamine HCl | 4 | mg |
| Riboflavin | 5 | mg |
| Niacinamide | 20 | mg |
| Pyridoxine HCl | 6 | mg |
| Folic Acid | 200 | mcg |
| Vitamin B-12 | 5 | mcg |
| Pantothenic Acid | 7.5 | mg |
| Biotin | 75 | mcg |
| Calcium (Carbonate & Citrate) | 12.5 | mg |
| Magnesium Oxide | 18.75 | mg |
| Iron (Fumarate) | 7.5 | mg |
| Zinc (Sulfate & Gluconate) | 7.5 | mg |
| Manganese Gluconate | 2 | mg |
| Selenium (L-Selanornethionine & Citrate) | 35 | mcg |
| Chromium Nicotinate | 37.5 | mcg |
| Copper Gluconate | 0.5 | mg |
| Green Tea Extract | 7.5 | mg |
| Grape Seed Extract | 7.5 | mg |
| N-Acetyl Glucosamine | 15 | mg |
| Citrus Bioflavanoids | 37.5 | mg |
| N-Acetyl Cysteine | 5 | mg |
| Sacred Lotus seed extract | 500 | mcg |
| S-adenosylmethlonine | 500 | mcg |
| Magnesium Stearate (lubricant) | 8.4 | mg |
| Stearic Acid (binder) | 42 | mg |
| Microcrystalline Cellulose (tablet aide) qs | 840 | mg |

### Procedure:

All of the above were mixed in a powder blender until completely homogenous. The mixed powder was fed into a tablet press and compressed into tablets.

A unique blend of protective antioxidants and essential nutrients with the entire Sacred Lotus seed (in food preparations the pithe (embryo) or lumule is removed due to bitter taste) used to promote good health and radiant skin is provided. This product may be taken, for example, twice per day.

### Reference Example 2

### FACE THERAPY

| | |
|---|---|
| Water | 50-75 |
| Carbomer 940 | 0.1-1 |
| Diazolidnyl Urea | 0.1-0.5 |
| Glycerine | 0.1-5 |
| Methylparaben | 0.1-0.3 |
| Panthol Powder | 0-1 |
| Triethanolamine | 0.5-2 |
| Stearic Acid | 0.5-5 |
| Glycol Stearate SE | 0.5-5 |
| Cetyl Alcohol | 0.5-4 |
| PPG-3 Myristal Ether | 0-3 |
| Isopropyl Myristate | 0.5-5 |
| Cocoa Butter | 0-3 |
| Rose Hip Seed Oil | 0-1 |
| Soybean Oil | 0-2 |
| Tocopheryl Acetate | 0-5 |
| Retinyl Palmitate | 0-1 |
| Propylparaben | 0.05-0.2 |
| Lecithin | 0-1 |
| Lipogard | 0-5 |
| Pregnenolene Acetate | 0-0.5 |
| Progesterona | 0-0.018 |
| Water | 0-10 |
| Glucosamine | 0-5 |
| Soy Isoflavones | 0-1 |
| Carnosine | 0-5 |
| Polygonum Cuspidatum | 0-1 |
| Propylene Glycol | 0-5 |
| Aloe Extract 10X | 0-5 |
| Ascorbyl Palmitate | 0-1 |
| Superoxide Dismutase | 0-2 |
| Fermalcase 1000 | 0-2 |
| Coneflower Extmct/Hydrocotyl extract | 0-5 |
| Green Tea Extract (GTP) | 0-5 |
| Grape Seed Extract | 0-5 |
| Pine Bark Extract | 0-5 |
| Lotus Seed Extract | 0-5 |
| Folate | 0-5 |
| Saccharide Isomerate | 0-5 |
| Ginseng Extract | 0-5 |
| Wild Yam Extract | 0-5 |
| Algea Extract | 0-5 |
| Tenox 6 | 0.05-0.8 |
| Vanilla 371B | 0-2 |
| Lycopene | 0-0.25 |
| Lutein | 0-0.25 |

### Procedure

The ingredients are mixed together using standard mixing techniques. A pleasant cream results.

### Reference Example 3

### LOTUS FIRMING SERUM

| | |
|---|---|
| Water | 50-85 |
| Panthenol Powder | 0-1 |
| Mg Ascorbyl Phosphate | 0-15 |
| Superoxide Dismutase | 0-2 |
| Catalase | 0-2 |
| Lotus Seed Extract | 0-15 |
| S-adenosylmethionine | 0-5 |
| Saccharide Isomerate | 0-6 |
| Na4EDTA | 0.01-0.5 |
| Glucosamine | 0-10 |
| Lipogard | 0-5 |
| Germaben II | 0.5-1.5 |
| Sodium Hyaluronate | 0-10 |
| Pine Bark Extract | 0-10 |
| Green Tea Extract | 0-10 |
| Coneflower Extract/Hydrocotyl extract | 0-10 |
| Vitamin A/D3 | 0-1.5 |
| Tocopheryl Acetate | 0-5 |
| Cyclomethicone | 0-10 |
| Sepigel 305 | 0-10 |
| Fragrance | 0-1 |
| Lutein | 0.001-0.5 |
| Lycopene | 0.001-0.5 |

### Procedure

The ingredients are mixed together using standard mixing techniques. A pleasant serum results.

### Reference Example 4

### ANTI-AGING MOISTURIZER

| | |
|---|---|
| Water | 50-75 |
| Carbomer 940 | 0.1-1 |
| Diazolidnyl Urea | 0.1-0.5 |
| Glycerine | 0-10 |
| Allantoin | 0-10 |
| Methylparaben | 0.1-0.3 |
| Panthenol Powder | 0.1-1 |
| Triethanolamine | 0.5-3 |
| Stearic Acid | 1-5 |
| Glycol Stearate SE | 1-5 |
| Cetyl Alcohol | 0.5-5 |
| PPG-3 Myristal Ether | 0.5-5 |
| Isopropyl Myristate | 0.1-5 |
| Cocoa Butter | 0-5 |
| Rose Hip Seed Oil | 0-1 |
| Soybean Oil | 0-2 |
| Tocopheryl Acetate | 0-5 |
| Retinyl Palmitate | 0-1 |
| Tocotrienol Oil | 0.001-0.5 |
| Tumeric Oil | 0-5 |
| CoQ10 | 0-0.5 |
| Propylparaben | 0.1-0.2 |
| Aloe Extract 10x | 0-5 |
| Beta-Carotene | 0-5 |
| Superoxide Dismutase | 0-2 |
| Fermalcase 1000 | 0-2 |
| Coneflower Extract/Hydrocotyl extract | 0-10 |
| Green Tea Extract (GTP20) | 0-10 |
| Grape Seed Extract | 0-10 |
| Pine Bark Extract | 0-10 |
| Lotus Seed Extract | 0-10 |
| S-adenosylmethionine | 0-5 |
| Ascorbyl Palmitate | 0-1 |
| Saccharide Isomerate | 0-5 |
| Ginkgo Biloba Extract | 0-10 |
| Ginseng Extract | 0-10 |
| Bilberry Extract | 0-10 |
| Rosemary Extract | 0-10 |
| Milk Thistle Extract | 0-10 |
| Olive leaf Extract | 0-10 |
| Tetrahydrodiferuloymethane | 0-5 |
| Carnosine | 0-5 |
| Alpha-Lipoic Acid | 0-1 |
| Water | 2-10 |
| Lutein | 0-0.25 |
| Lycopene | 0-0.25 |

### Procedure

The Ingredients are mixed together using standard mixing techniques.

### Reference Example 5

### EYE THERAPY

| | |
|---|---|
| Water | 20-65 |
| Glycerin | 0-5 |
| NA4 EDTA | 0-0.5 |
| Panthenol | 0-1 |
| Diazolidinyl Urea | 0.1-0.5 |
| Methylparaben | 0.1-0.3 |
| Tween 20 | 0-2 |
| Veegum 5% | 10-20 |
| Sunflower Oil | 5-15 |
| Mineral Oil | 5-15 |
| Arlacel 165 | 1-5 |
| Cetyl Alcohol | 1-5 |
| Phenyl Dimethicone | 1-5 |
| Olive Oil | 0-5 |
| Propylparaben | 0.05-0.2 |
| Tocopheryl Acetate | 0-5 |
| 2 POE Stearyl Stearate | 0-5 |
| Lecithin | 0-2 |
| Glyceryl Dilaurate | 0-2 |
| Sodium Lauryl Sulfate | 0-0.5 |
| Retinyl Palmitate | 0-2 |
| Magnesium Ascorb Phos | 0-5 |
| Fermocalase 1000 | 0-2 |
| Superoxide Dismutase BT | 0-2 |
| Aloe 10X | 0-5 |
| Algae Extract APT | 0-5 |
| Hydro Soy Protien | 0-5 |
| Coneflower Extract/Hydrocotyl extract | 0-8 |
| Glucosamine | 0-5 |
| Lotus Seed Extract | 0-10 |
| Trimethylglycine | 0-5 |
| Polygonum Cuspidatum | 0-0.5 |
| Propylene Glycol | 0-5 |
| Wild Yam Extract | 0-5 |
| Glycosaminoglycans | 0-10 |
| Pregnenolone Liposome | 0-2 |
| Progesteone Liposome | 0-2 |
| Soy Isoflavones | 0-0.3 |
| Squalane Ubiquinone | 0-5 |
| Tenox 6 | 0.05-0.5 |
| Lycopene | 0.001-0.8 |
| Lutein | 0.001-0.3 |
| Sodium Hydroxide 25% | 0-5 |

### Procedure

The ingredients are mixed together using standard mixing techniques.

## Claims

1. An oral or a topical composition comprising:
a) Lotus seed extract;
b) Lotus flower extract;
c) one or more methyl donors; and
d) a compatible vehicle for oral or topical application.

2. An oral or a topical composition as claimed in claim 1, which includes extract of the Sacred Lotus (*Nelumbo nucifera*)

3. An oral or topical composition as claimed in claim 1 or claim 2, which includes L-isoaspartyl methyltransferase from Lotus extract.

4. An oral or topical composition as claimed in any one of claims 1 to 3, wherein said one or more methyl donors are selected from the group consisting of methionine, betain, dimethylglycine, choline, polyenyl phosphatidylcholine, phosphatidylserine, phosphatidyl choline, glycerylphosphoryl choline, and S-adenosyl-L-methionine.

5. An oral or topical composition as claimed in any one of claims 1 to 4, further comprising one or more vitamins.

6. An oral or topical composition as claimed in any one of claims 1 to 5, further comprising one or more minerals.

7. An oral or topical composition as claimed in any one of claims 1 to 6, further comprising one or more antioxidants.

8. An oral or topical composition as claimed in any one of claims 1 to 7, further comprising one or more amino acids.

9. An oral or topical composition as claimed in any one of claims 1 to 8, further comprising one or more hormones.

## Patentansprüche

1. Orale oder topische Zusammensetzung umfassend:
a) Lotussamenextrakt;
b) Lotusblütenextrakt;
c) einen oder mehrere Methyldonoren; und
d) einen passenden Trägerstoff für die orale oder topische Applikation.

2. Orale oder topische Zusammensetzung wie in Anspruch 1 beansprucht, welche einen Extrakt der Sacred Lotus (Nelumbo nucifera) enthält.

3. Orale oder topische Zusammensetzung wie in Anspruch 1 oder Anspruch 2 beansprucht, welche L-Isoaspartylmethyltransferase vom Lotusextrakt enthält.

4. Orale oder topische Zusammensetzung wie in einem der Ansprüche 1 bis 3 beansprucht, wobei genannte ein oder mehrere Methyldonoren ausgewählt sind aus der Gruppe bestehend aus Methionin, Betain, Dimethylglycin, Cholin, Polyenyl-Phosphatidylcholin, Phosphatidylserin, Phosphatidylcholin, Glyceryl-Phosphoryl-Cholin und S-Adenosyl-L-Methionin.

5. Orale oder topische Zusammensetzung wie in einem der Ansprüche 1 bis 4 beansprucht, weiter umfassend ein oder mehrere Vitamine.

6. Orale oder topische Zusammensetzung wie in einem der Ansprüche 1 bis 5 beansprucht, weiter umfassend ein oder mehrere Mineralien.

7. Orale oder topische Zusammensetzung wie in einem der Ansprüche 1 bis 6 beansprucht, weiter umfassend eine oder mehrere Antioxidantien.

8. Orale oder topische Zusammensetzung wie in einem der Ansprüche 1 bis 7 beansprucht, weiter umfassend eine oder mehrere Aminosäuren.

9. Orale oder topische Zusammensetzung wie in einem der Ansprüche 1 bis 8 beansprucht, weiter umfassend ein oder mehrere Hormone.

## Revendications

1. Composition orale ou topique comprenant :
a) un extrait de graines de Lotus ;
b) un extrait de fleurs de Lotus ;
c) un ou plusieurs donneurs de méthyle ; et
d) un véhicule compatible pour l'application orale ou topique.

2. Composition orale ou topique selon la revendication 1, qui comprend un extrait de Lotus sacré (*Nelumbo nucifera*).

3. Composition orale ou topique selon la revendication 1 ou la revendication 2, qui comprend de la L-isoasparyl-méthyltransférase provenant d'extraits de Lotus.

4. Composition orale ou topique selon l'une quelconque des revendications 1 à 3, dans laquelle les un ou plusieurs donneurs de méthyle sont choisis parmi le groupe composé de la méthionine, de la bétaïne, de la diméthylglycine, de la choline, de la polyénylphosphatidylcholine, de la phosphatidylsérine, de la phosphatidylcholine, de la glycérylphosphorylcholine et de la S-adénosyl-L-méthionine.

5. Composition orale ou topique selon l'une quelconque des revendications 1 à 4, comprenant en outre une ou plusieurs vitamines.

6. Composition orale ou topique selon l'une quelconque des revendications 1 à 5, comprenant en outre un ou plusieurs minéraux.

7. Composition orale ou topique selon l'une quelconque des revendications 1 à 6, comprenant en outre un ou plusieurs anti-oxydants.

8. Composition orale ou topique selon l'une quelconque des revendications 1 à 7, comprenant en outre un ou plusieurs acides aminés.

9. Composition orale ou topique selon l'une quelconque des revendications 1 à 8, comprenant en outre une ou plusieurs hormones.
